# EUROPEAN PATENT SPECIFICATION

(11) **EP 2 718 702 B1**
(45) Date of publication and mention of the grant of the patent: **30.11.2016**
(21) Application number: 12796632.3
(22) Date of filing: 05.06.2012
(51) Int. Cl.: G01N 25/04, B60K 15/03, F02M 37/22, G01N 33/28, F02M 31/125, F02M 15/04, F02M 15/02, F02M 31/18

(54) **METHOD AND SYSTEM FOR DETECTING WAXING OF DIESEL FUEL IN A FUEL TANK IN A MOTOR VEHICLE**
VERFAHREN UND SYSTEM ZUR ERKENNUNG DER PARAFFINIERUNG EINES DIESELKRAFTSTOFFS IM EINEN KRAFTSTOFFTANK EINES KRAFTFAHRZEUGS
PROCÉDÉ ET SYSTÈME PERMETTANT DE DÉTECTER L'APPARITION DE CIRE DANS LE CARBURANT DIESEL SE TROUVANT DANS LE RÉSERVOIR À CARBURANT D'UN VÉHICULE AUTOMOBILE

(30) Priority: 09.06.2011 SE 1150525
(43) Date of publication of application: 16.04.2014
(73) Proprietor: Scania CV AB, 151 87 Södertälje (SE)
(72) Inventor: ERIKSSON, Henrik, SE-152 41 Södertälje (SE); ROOS, Stefan, S-830 10 Undersåker (SE)
(74) Representative: Scania CV AB
(86) International application number: PCT/SE2012/050600
(87) International publication number: WO 2012/169957

(56) References cited:
- EP-A1- 0 259 252
- EP-A1- 1 900 915
- WO-A1-2009/008408
- CN-U- 201 665 212
- FR-A- 1 453 943
- GB-A- 1 029 165
- US-A- 4 770 540
- US-A1- 2003 193 989
- US-A1- 2005 053 116
- US-A1- 2010 012 410

## Description

### FIELD OF THE INVENTION AND PRIOR ART

The present invention relates to a method and a system for detecting waxing of diesel fuel in a fuel tank in a motor vehicle. The invention relates also to a computer programme and a computer programme product comprising computer programme code for implementing a method according to the invention, and to an electronic control unit.

When its temperature drops to a certain level, diesel fuel becomes subject to waxing, which means that wax crystals form in it. The temperature at which this waxing commences is referred to as the fuel's waxing point. In a diesel-powered vehicle the wax crystals which form in its fuel may be trapped in the fuel filter situated between the fuel tank and the engine. This leads to the filter being obstructed by the wax crystals and no longer being able to allow necessary amounts of fuel to reach the engine, making it difficult or completely impossible for the engine to run. A known way of preventing such obstruction of the fuel filter in a motor vehicle is to activate various kinds of electrically powered fuel heaters when the temperature of the fuel drops to a certain predetermined level, e.g. as described in US 2003/0116490 A1 and EP 0 412 210 A1. This warming of the fuel counteracts its waxing. As fuel heaters consume energy when switched on, and thereby cause increased fuel consumption of the vehicle, it is desirable not to have them switched on unnecessarily but only when there is real need.

The waxing point of diesel fuel depends inter alia on various kinds of additives added to the fuel to give it a suitable waxing point. A problem with the abovementioned solution with fuel heaters activated when the fuel is at a certain temperature level is that not only is the waxing point not declared by fuel suppliers but it may also vary from one refuelling to another. The vehicle's driver therefore usually has no accurate knowledge of the prevailing waxing point of the fuel contained in its fuel tank at the time. This means that the temperature level for activation of fuel heaters has to be chosen with a relatively large safety margin which may lead to their being activated more often than is really necessary. There is of course also a risk of the prevailing waxing point of the fuel in the tank being significantly higher than expected, potentially leading to heaters not being activated in time to prevent waxing of the fuel and obstruction of the vehicle's fuel filter.

An alternative solution which makes it possible to prevent these latter problems is based on waxing of the diesel fuel being detected on board the vehicle by an optical method, e.g. as described in US 2005/0053116 A1. That solution does however involve relatively complex and expensive equipment.

In a laboratory, the cloud point of a hydrocarbon liquid can be determined using two temperature sensors at two different locations inside the container holding the liquid sample, because convection is hindered once waxing starts to occur; this is known e.g. from FR 1453943 A.

### OBJECT OF THE INVENTION

The object of the present invention is to propose a novel and advantageous way of detecting waxing of diesel fuel in a fuel tank in a motor vehicle.

### SUMMARY OF THE INVENTION

According to the present invention, said object is achieved by a method with the features defined in claim 1 and a system with the features defined in claim 5.

### According to the invention,

- measured values from temperature sensors are used to generate a first temperature value which represents the temperature of the diesel fuel in a first region of the fuel tank, and a second temperature value which represents the temperature of the diesel fuel in a second region of the tank,
- a differential value representing the prevailing difference between the first temperature value and the second temperature value is determined, and
- the differential value is compared with a limit value for detection of waxing of the diesel fuel in the tank.

When the temperature of the diesel fuel in the fuel tank drops, heat will be released from the fuel via the sidewalls and bottom of the tank and via the fuel surface. The gradual temperature decrease at the edges of the fuel volume in the fuel tank leads to density differences in the fuel, resulting in circulation of the fuel in the tank and heat transfer by natural convection. So long as the temperature of the fuel in the tank is above its waxing point, the natural convection will lead to equalisation of its temperature in the tank. The natural convection thus counteracts temperature stratification of the fuel in the tank, which means that there will be no obvious fuel temperature differences in different parts of the tank. When the fuel in the tank reaches a low enough temperature to become subject to waxing, the fuel circulation in the tank and the effectiveness of the natural convection are hindered, so the temperature equalisation will decrease, leading to more obvious fuel temperature differences in different parts of the tank. By registering such temperature differences it therefore becomes possible to detect waxing of the fuel in the tank. The solution according to the invention provides a simple way of detecting waxing of diesel fuel in a fuel tank without having to use other than relatively simple and inexpensive temperature sensors. When waxing of the fuel is detected, a warning signal or a message which indicates the waxing may for example be generated. It is also possible to have one or more fuel heaters activated to warm the fuel when its waxing is detected.

Other advantageous features of the method and system according to the invention are indicated by the independent claims and the description set out below.

The invention relates also to a computer programme with the features defined in claim 9, a computer programme product with the features defined in claim 10 and an electronic control unit with the features defined in claim 11.

### BRIEF DESCRIPTION OF THE DRAWINGS

The invention is described below in more detail on the basis of embodiment examples with reference to the attached drawings, in which
- Fig. 1: is a schematic diagram of a motor vehicle with a combustion engine and a system according to the present invention for detecting waxing of diesel fuel in the vehicle's fuel tank,
- Fig. 2: is a schematic diagram of an electronic control unit for implementing a method according to the invention, and
- Fig. 3: is a flowchart illustrating a method according to an embodiment of the invention.

### DETAILED DESCRIPTION OF EMBODIMENTS OF THE INVENTION

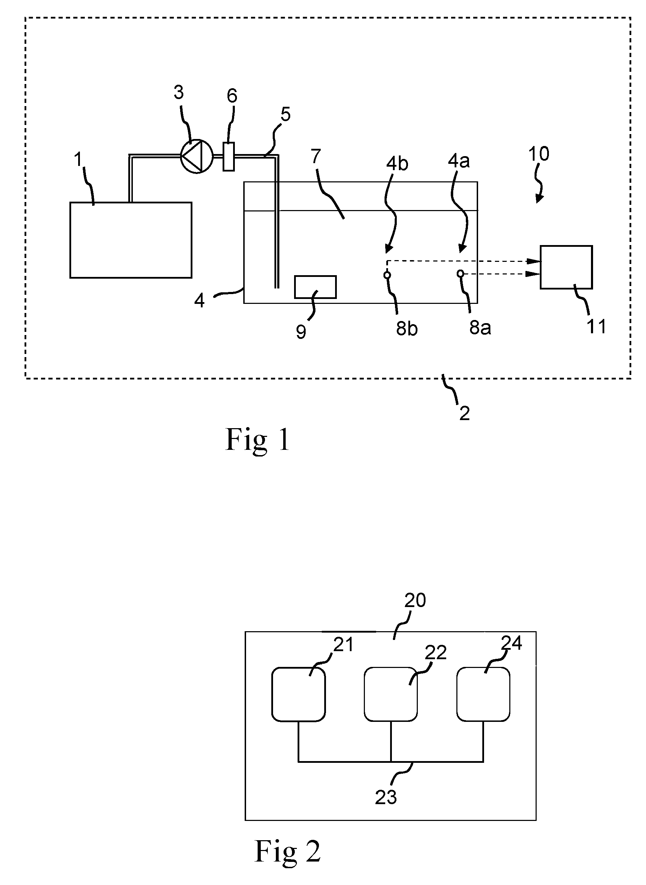

Fig. 1 depicts schematically a combustion engine 1 of a motor vehicle 2. A pump 3 is provided to feed diesel fuel to the engine 1 from a fuel tank 4 via a fuel line 5. A fuel filter 6 is provided in the fuel line 5 to filter the diesel fuel. It is also possible to use two or more fuel filters to filter the diesel fuel, e.g. a first fuel filter situated in the fuel line 5 between the fuel tank 4 and the pump 3 and a second fuel filter situated in the fuel line between the pump 3 and the engine 1.

Fig. 1 depicts schematically a system 10 according to the present invention for detecting waxing of the diesel fuel 7 contained in the vehicle's fuel tank 4.

One or more first temperature sensors 8a are provided to measure the temperature of the diesel fuel in a first region 4a of the fuel tank 4, and one or more second temperature sensors 8b to measure the temperature of the diesel fuel in a second region 4b of the tank. In the embodiment depicted in Fig. 1, said first and second regions 4a, 4b of the tank are situated at a distance from one another in a horizontal direction but might alternatively be at a distance from one another in a vertical direction or in both horizontal and vertical directions.

The system 10 according to the invention comprises an electronic data processing device 11 connected to said temperature sensors 8a, 8b in order to receive from them measured values for the temperature of the diesel fuel 7 in said first and second regions 4a, 4b of the fuel tank.

The electronic data processing device 11 is adapted to using measured values from said temperature sensors 8a, 8b to generate a first temperature value V_{T1} which represents the temperature of the fuel in said first region 4a of the fuel tank, and a second temperature value V_{T2} which represents the temperature of the fuel in said second region 4b of the tank. The data processing device 11 is further adapted to determining a differential value V_{diff} which represents the prevailing difference between the first temperature value V_{T1} and the second temperature value V_{T2}, and to comparing this differential value V_{diff} with a limit value V_{G} for detection of waxing of the fuel. The differential value V_{diff} is with advantage calculated as the difference V_{diff=} V_{T1}-V_{T2} or the difference V_{diff}= V_{T2}-V_{T1}, and waxing of the fuel is detected when it is found that the differential value V_{diff} has an absolute value greater than or equal to the limit value V_{G}.

The limit value V_{G} is chosen such as to be somewhat greater than the small fuel temperature differences which may normally occur between the two regions 4a, 4b of the tank 4 before the temperature of the fuel drops low enough for waxing to occur.

When the data processing device 11 detects waxing, i.e. when the aforesaid comparison between the differential value V_{diff} and the limit value V_{G} shows that the diesel fuel 7 in the tank 4 has undergone waxing, the data processing device 11 is adapted to activating one or more fuel heaters 9 to warm the fuel. Warming the fuel by means of said fuel heaters counteracts continued waxing of the fuel and dissolves the wax crystals already formed. In the example depicted, the heater 9 is situated within the tank 4. As an alternative or in addition to the heater 9 situated in the tank, fuel heaters might be provided on the outside of the tank, to warm from outside the fuel 7 contained in the tank, and/or be provided in the fuel filter 6 and/or in the fuel line 5 between the tank and the filter. Each fuel filter would with advantage be powered electrically and might for example comprise one or more electrical heating elements.

When waxing is detected, the data processing device 11 may also be adapted to generating a warning signal, e.g. in the form of a visual or acoustic signal, or a message which indicates waxing of the fuel. A driver in the vehicle 2 may thus for example be made aware that the fuel has undergone waxing.

The aforesaid data processing device 11 may be implemented by means of a single electronic control unit of the vehicle 2, as depicted in Fig. 1, but might alternatively be implemented by means of two or more mutually cooperating electronic control units of the vehicle.

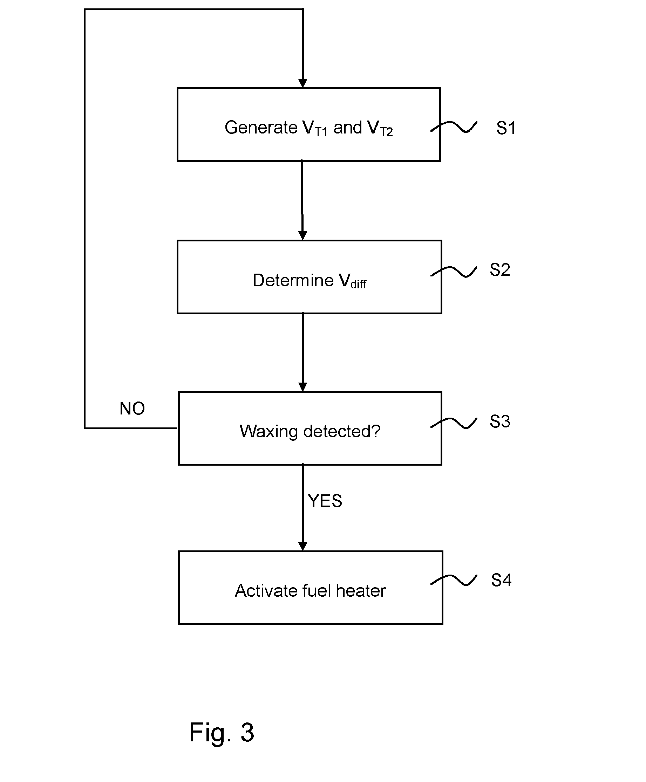

Fig. 3 is a flowchart illustrating an embodiment of a method according to the present invention for detecting waxing of diesel fuel 7 in a fuel tank 4 in a motor vehicle 2.
As a first step S1, measured values from temperature sensors 8a, 8b are used to generate a first temperature value VT₁ which represents the temperature of the fuel 7 in a first region 4a of the fuel tank, and a second temperature value V_{T2} which represents the temperature of the fuel in a second region 4b of the tank.
As a second step S2, a differential value V_{diff} representing the prevailing difference between the first temperature value V_{T1} and the second temperature value V_{T2} is determined.

As a third step S3, the differential value V_{diff} is compared with a limit value V_{G} for detection of waxing of the fuel in the fuel tank.
If the comparison at step S3 shows that the fuel has not undergone waxing, steps S1-S3 are repeated after a certain period of time.
If the comparison at step S3 shows that the fuel has undergone waxing, one or more fuel heaters for warming the fuel are activated as a fourth step S4.

Computer programme code for implementing a method according to the invention is with advantage included in a computer programme which can be read into the internal memory of a computer, e.g. the internal memory of an electronic control unit of a motor vehicle. Such a computer programme is with advantage provided via a computer programme product comprising a data storage medium which can be read by a computer and which has the computer programme stored on it. Said data storage medium is for example an optical data storage medium in the form of a CD ROM disc, a DVD disc etc., a magnetic data storage medium in the form of a hard disc, a diskette, a cassette tape etc., or a flash memory or a memory of the ROM, PROM, EPROM or EEPROM type.

Fig. 2 illustrates very schematically an electronic control unit 20 comprising an execution means 21, e.g. a central processor unit (CPU), for execution of computer software. The execution means 21 communicates with a memory 22, e.g. of the RAM type, via a data bus 23. The control unit 20 comprises also a data storage medium 24, e.g. in the form of a flash memory or a memory of the ROM, PROM, EPROM or EEPROM type. The execution means 21 communicates with the data storage medium 24 via the data bus 23. A computer programme comprising computer programme code for implementing a method according to the invention, e.g. in accordance with any of the embodiments illustrated in Figs. 3 and 4, is stored on the data storage medium 24.

The invention is of course in no way restricted to the embodiments described above, since many possibilities for modifications thereof are likely to be obvious to one skilled in the art without therein having to deviate from the invention's basic concept such as defined in the attached claims.

## Claims

1. A method for detecting waxing of diesel fuel inside a fuel tank (4) within a motor vehicle (2), **characterised in that** the method comprises the following steps:
- using measured values from temperature sensors (8a, 8b) to generate a first temperature value (V_{T1}) which represents the temperature of the fuel in a first region (4a) of the fuel tank, and a second temperature value (V_{T2}) which represents the temperature of the fuel in a second region (4b) of the tank,
- determining a differential value (V_{diff}) which represents the prevailing difference between the first temperature value (V_{T1}) and the second temperature value (V_{T2}), and
- comparing the differential value (V_{diff}) with a limit value (V_{G}) for detection of waxing of the fuel in the tank (4).

2. A method according to claim 1, **characterised in that** waxing of the fuel in the tank (4) is detected when it is found that the differential value (V_{diff}) has an absolute value greater than or equal to the limit value (V_{G}).

3. A method according to claim 1 or 2, **characterised in that** a warning signal or a message which indicates waxing of the diesel fuel is generated when such waxing is detected.

4. A method according to any one of claims 1-3, **characterised in that** one or more fuel heaters (9) are activated to warm the fuel when waxing of the fuel is detected.

5. A system for detecting waxing of diesel fuel in a fuel tank in a motor vehicle, **characterised**
- **in that** the system (10) comprises an electronic data processing device (11) adapted to using measured values from temperature sensors to generate a first temperature value (V_{T1}) which represents the temperature of the fuel in a first region of the tank, and a second temperature value (V_{T2}) which represents the temperature of the fuel in a second region of the tank,
- that the data processing device (11) is adapted to determining a differential value (V_{diff}) which represents the prevailing difference between the first temperature value (V_{T1}) and the second temperature value (V_{T2}), and to comparing this differential value (V_{diff}) with a limit value (V_{G}) for detection of waxing of the fuel in the tank.

6. A system according to claim 5, **characterised in that** the data processing device (11) is adapted to detecting waxing of the fuel in the tank when it finds that the differential value (V_{diff}) has an absolute value greater than or equal to the limit value (V_{G}).

7. A system according to claim 5 or 6, **characterised in that** the data processing device (11) is adapted to generating a warning signal or a message which indicates waxing of the fuel when it detects such waxing.

8. A system according to any one of claims 5-7, **characterised in that** the data processing device (11) is adapted to activating one or more fuel heaters to warm the fuel when it detects waxing of the fuel.

9. A computer programme comprising computer programme code for causing a computer to implement a method according to any one of claims 1-4 when the computer programme is executed on the computer.

10. A computer programme product comprising a data storage medium which can be read by a computer and on which the programme code of a computer programme according to claim 9 is stored.

11. An electronic control unit of a motor vehicle comprising an execution means (21), a memory (22) connected to the execution means and a data storage medium (24) which is connected to the execution means and on which the computer programme code of a computer programme according to claim 9 is stored.

## Patentansprüche

1. Verfahren zum Detektieren des Paraffinierens von Dieselkraftstoff im Innern eines Kraftstofftanks (4) innerhalb eines Kraftfahrzeugs (2), **dadurch gekennzeichnet, dass** das Verfahren die folgenden Schritte umfasst:
- Benutzen von Messwerten von Temperatursensoren (8a, 8b), um einen ersten Temperaturwert (V_{T1}), der die Temperatur des Kraftstoffes in einem ersten Bereich (4a) des Kraftstofftanks darstellt, und einen zweiten Temperaturwert (V_{T2}), der die Temperatur des Kraftstoffes in einem zweiten Bereich (4b) des Tanks darstellt, zu generieren,
- Bestimmen eines Differenzwertes (V_{diff}), der den zwischen dem ersten Temperaturwert (V_{T1}) und dem zweiten Temperaturwert (V_{T2}) herrschenden Unterschied darstellt, und
- Vergleichen des Differenzwertes (V_{diff}) mit einem Grenzwert (V_{G}) zum Detektieren des Paraffinierens des Kraftstoffes im Tank (4).

2. Verfahren nach Anspruch 1, **dadurch gekennzeichnet, dass** das Paraffinieren des Kraftstoffes im Tank (4) detektiert wird, wenn festgestellt wird, dass der Differenzwert (V_{diff}) einen absoluten Wert hat, der größer als oder gleich dem Grenzwert (V_{G}) ist.

3. Verfahren nach Anspruch 1 oder 2, **dadurch gekennzeichnet, dass** ein Warnsignal oder eine Nachricht generiert wird, die das Parafhnieren des Dieselkraftstoffs anzeigt, wenn ein solches Paraffinieren detektiert wird.

4. Verfahren nach einem der Ansprüche 1-3, **dadurch gekennzeichnet, dass** ein oder mehrere Kraftstoffheizungen (9) aktiviert werden, um den Kraftstoff aufzuwärmen, wenn das Paraffinieren des Kraftstoffs detektiert wird.

5. Ein System zum Detektieren des Paraffinierens von Dieselkraftstoff in einem Kraftstofftank in einem Kraftfahrzeug, **dadurch gekennzeichnet,**
- **dass** das System (10) ein elektronisches Datenverarbeitungsgerät (11) umfasst, das dazu geeignet ist, Messwerte von Temperatursensoren zu benutzen, um einen ersten Temperaturwert (V_{T1}), der die Temperatur des Kraftstoffes in einem ersten Bereich des Tanks darstellt, und einen zweiten Temperaturwert (V_{T2}), der die Temperatur des Kraftstoffes in einem zweiten Bereich des Tanks darstellt, zu generieren,
- **dass** das Datenverarbeitungsgerät (11) dazu geeignet ist, einen Differenzwert (V_{diff}) zu bestimmen, der den zwischen dem ersten Temperaturwert (V_{T1}) und dem zweiten Temperaturwert (V_{T2}) herrschenden Unterschied darstellt, und den Differenzwert (V_{diff}) mit einem Grenzwert (V_{G}) zur Detektion des Paraffinierens des Kraftstoffes im Tank zu vergleichen.

6. System nach Anspruch 5, **dadurch gekennzeichnet, dass** das Datenverarbeitungsgerät (11) dazu geeignet ist, das Paraffinieren des Kraftstoffs im Tank zu detektieren, wenn es feststellt, dass der Differenzwert (V_{diff}) einen absoluten Wert hat, der größer als oder gleich dem Grenzwert (V_{G}) ist.

7. System nach Anspruch 5 oder 6, **dadurch gekennzeichnet, dass** das Datenverarbeitungsgerät (11) dazu geeignet ist, ein Warnsignal oder eine Nachricht zu generieren, das das Paraffinieren des Kraftstoffs anzeigt, wenn es ein solches Paraffinieren detektiert.

8. System nach einem der Ansprüche 5-7, **dadurch gekennzeichnet, dass** das Datenverarbeitungsgerät (11) dazu geeignet ist, ein oder mehrere Kraftstoffheizungen zu aktivieren, um den Kraftstoff aufzuwärmen, wenn es das Paraffinieren des Kraftstoffs detektiert.

9. Computerprogramm, das Computerprogrammcode umfasst, um einen Computer zu veranlassen, ein Verfahren nach einem der Ansprüche 1-4 zu implementieren, wenn das Computerprogramm auf dem Computer ausgeführt wird.

10. Computerprogrammprodukt, das ein Datenspeichermedium umfasst, das von einem Computer gelesen werden kann und auf dem der Programmcode eines Computerprogramms nach Anspruch 9 gespeichert ist.

11. Elektronische Steuereinheit eines Kraftfahrzeugs, das umfasst: ein Ausführungsmittel (21), einen Speicher (22), der mit dem Ausführungsmittel verbunden ist, und ein Datenspeichermedium (24), das mit dem Ausführungsmittel verbunden ist und auf dem der Computerprogrammcode eines Computerprogramms nach Anspruch 9 gespeichert ist.

## Revendications

1. Procédé de détection d'un paraffinage d'un carburant diesel à l'intérieur d'un réservoir de carburant (4) dans un véhicule à moteur (2), **caractérisé en ce que** le procédé comprend les étapes suivantes :
- utilisation des valeurs mesurées par des capteurs de température (8a, 8b) pour générer une première valeur de température (V_{T1}) qui représente la température du carburant dans une première région (4a) du réservoir de carburant, et une deuxième valeur de température (V_{T2}) qui représente la température du carburant dans une deuxième région (4b) du réservoir,
- détermination d'une valeur différentielle (V_{diff}) qui représente la différence qui prévaut entre la première valeur de température (V_{T1}) et la deuxième valeur de température (V_{T2}), et
- comparaison de la valeur différentielle (V_{diff}) à une valeur limite (V_{G}) pour la détection d'un paraffinage du carburant dans le réservoir (4).

2. Procédé selon la revendication 1, **caractérisé en ce qu'**un paraffinage du carburant dans le réservoir (4) est détecté lorsqu'il est trouvé que la valeur différentielle (V_{diff}) a une valeur absolue supérieure ou égale à la valeur limite (V_{G}).

3. Procédé selon la revendication 1 ou 2, **caractérisé en ce qu'**un signal d'avertissement ou un message indiquant un paraffinage du carburant diesel est généré lorsqu'un tel paraffinage est détecté.

4. Procédé selon l'une quelconque des revendications 1 à 3, **caractérisé en ce qu'**un ou plusieurs réchauffeurs de carburant (9) sont activés pour chauffer le carburant lorsqu'un paraffinage du carburant est détecté.

5. Système de détection d'un paraffinage d'un carburant diesel dans un réservoir de carburant dans un véhicule à moteur, **caractérisé :**
- **en ce que** le système (10) comprend un dispositif électronique de traitement de données (11) adapté pour utiliser des valeurs mesurées par des capteurs de température pour générer une première valeur de température (V_{T1}) qui représente la température du carburant dans une première région du réservoir, et une deuxième valeur de température (V_{T2}) qui représente la température du carburant dans une deuxième région du réservoir,
- **en ce que** le dispositif de traitement de données (11) est adapté pour déterminer une valeur différentielle (V_{diff}) qui représente la différence qui prévaut entre la première valeur de température (V_{T1}) et la deuxième valeur de température (V_{T2}), et pour comparer cette valeur différentielle (V_{diff}) à une valeur limite (V_{G}) pour la détection d'un paraffinage du carburant dans le réservoir.

6. Système selon la revendication 5, **caractérisé en ce que** le dispositif de traitement de données (11) est adapté pour détecter un paraffinage du carburant dans le réservoir lorsqu'il est trouvé que la valeur différentielle (V_{diff}) a une valeur absolue supérieure ou égale à la valeur limite (V_{G}).

7. Système selon la revendication 5 ou 6, **caractérisé en ce que** le dispositif de traitement de données (11) est adapté pour générer un signal d'avertissement ou un message indiquant un paraffinage du carburant lorsqu'il détecte un tel paraffinage.

8. Système selon l'une quelconque des revendications 5 à 7, **caractérisé en ce que** le dispositif de traitement de données (11) est adapté pour activer un ou plusieurs réchauffeurs de carburant pour chauffer le carburant lorsqu'il détecte un paraffinage du carburant.

9. Programme informatique comprenant un code de programme informatique destiné à amener un ordinateur à mettre en oeuvre un procédé selon l'une quelconque des revendications 1 à 4, lorsque le programme informatique est exécuté sur l'ordinateur.

10. Produit de programme informatique comprenant un support de stockage de données qui peut être lu par un ordinateur et sur lequel est stocké le code de programme d'un programme informatique selon la revendication 9.

11. Unité de commande électronique d'un véhicule à moteur comprenant des moyens d'exécution (21), une mémoire (22) connectée aux moyens d'exécution et un support de stockage de données (24) qui est connecté aux moyens d'exécution et sur lequel est stocké le code de programme informatique d'un programme informatique selon la revendication 9.
